# EUROPEAN PATENT APPLICATION

(11) **EP 4 700 037 A1**
(43) Date of publication of application: **25.02.2026**
(21) Application number: 24195363.7
(22) Date of filing: 20.08.2024
(51) Int. Cl.: C07J 9/00

(54) **SYNTHESIS OF ISOTOPE LABELED CHOLESTEROL DERIVATIVES AS DIAGNOSTIC AGENTS**

(71) Applicant: Otto-von-Guericke-Universität Magdeburg Körperschaft des Öffentlichen Rechts, 39106 Magdeburg (DE); Universitätsklinikum Jena Körperschaft des öffentlichen Rechts, 07747 Jena (DE)
(72) Inventor: SCHINZER, Dieter, 38108 Braunschweig (DE); SPIESS, Oliver, 21029 Hamburg (DE); BAUER, Michael, 07749 Jena (DE); PRESS, Adrian Tibor, 07749 Jena (DE); MEHMETAJ, Klea, 07747 Jena (DE)
(74) Representative: Braeuning Schubert Patentanwälte GbR

(57) **Abstract**

The invention concerns synthesis of isotope labeled cholesterol derivatives as diagnostic agents. The isotopes are radio labeled and/or labelled isotopes, namely ¹⁸F, and/or ¹³¹I, and/or ¹²⁴I , and/or deuterium. In addition, the invention concerns the use of radiolabeled cholesterol derivatives as well as their physiologically tolerated salts for the preparation of pharmaceutical drugs for the treatment of infectious diseases, particularly pneumonia or septic cardiomyopathy, but also other diseases associated with decreased cholesterol levels (hypercholesterinemia), e.g. Smith-Lemli-Opitz syndrome. In a further addition, the invention discloses pharmaceutical formulation containing radiolabeled cholesterol derivatives as well as their physiologically tolerated salts for the treatment of infectious diseases, particularly pneumonia or septic cardiomyopathy, but also other diseases associated with decreased cholesterol levels (hypercholesterinemia), e.g. Smith-Lemli-Opitz syndrome.

## Description

The invention concerns synthesis of isotope labeled cholesterol derivatives as diagnostic agents. The isotopes are radio labeled and/or labelled isotopes, namely ¹⁸F, and/or ¹³¹I, and/or ¹²⁴I, and/or deuterium.

Cholesterol is essential for multiple cellular processes. Changes in cellular cholesterol can alter physiological properties, making cells resistant to bacterial virulence factors such as S. pneumoniae Pneumolysin, which is crucial for pathogenesis and infection. However, the rapid metabolization of cholesterol limits its therapeutic use. This patent introduces novel multimodal compounds that have superior stability due to chemical modifications, enhancing their ability to counteract infections. Additionally, their radiolabels allow detection via positron emission tomography (PET) combined with computed tomography (CT), enabling personalized approaches and drug monitoring in individual patients. These compounds also serve as novel cholesterol-replacing additives for both established and new nanomedicines.

The object of the present invention is to prepare isotope labeled and/or radio labeled cholesterol derivatives which have improved pharmacokinetic and/or pharmacodynamic properties when compared with compounds already known.

It has now been found surprisingly that the deuterated substituted cholesterol derivatives according to the invention have essentially better pharmacokinetic and/or pharmacodynamic properties than the undeuterated and/or unlabeled radio active compounds.

According to the invention the object is thus solved by the preparation of deuterated and/or radiolabeled cholesterol derivatives of the general formula I 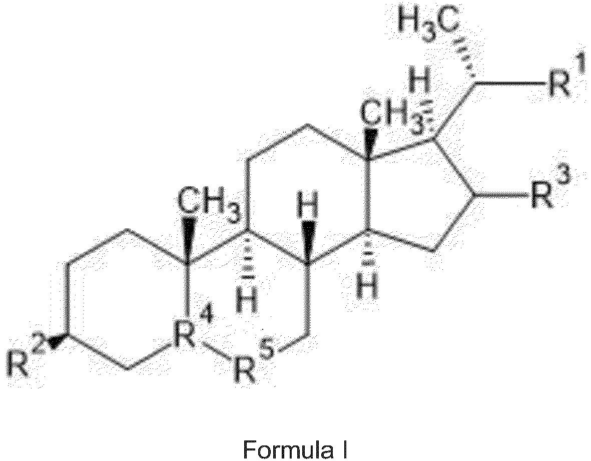 wherein
- R¹ is: -CH=CH₂,.-CH₂-CHX₂, -CH₂-CH₂-CH₂-CH(CH₃)₂, -CH₂-CH₂-CH₂-CH(CH₃)(CH₂X), -CH₂-CH₂-CH₂-CH(CH₃)(CH ₂OCH₃), -CH₂-CH₂-CH₂-CH(CH₃)(CH ₂OH), -CH₂-CH₂-CH(CH₂CH₃)(CH(CH₃)₂), -CHF-CH₂-CH(CH₂CH₃)(CH(CH₃)₂), or -CH₂-CHX-CH(CH₂CH₃)(CH(CH₃)₂),
- R² is: -OH, -OCH₃, -O-CO-CH₂OH, -O-CO-CH₂X, -O-CO-CHX₂, -O-CO-CX₃, or stealth polymers like polyethylene glycol (PEG), Poly(ethylene glycol) monomethyl ether (mPEG), Polyoxazolines (Pox), betaines and others,
- R³ is: -H, -F, -I, -D, -OH, or -OCH₃
- R⁴ is: -CH or -CD,
- R⁵ is: -CH₂, -CHD or -CD₂,
- R⁴ and R⁵: is -CH= CH₂-, -CH= CD₂-, -CH=CHD-, -CD=CH₂, -CD=CD₂- or -CD=CHD-,
- X is: F or I or D,
and wherein at least one of the groups R¹ to R³ contains radio labeled and/or labelled isotopes, namely ¹⁸F, and/or ¹³¹I, and/or ¹²⁴I, and/or deuterium isotope labeled derivatives.

The following isotope labeled cholesterol derivatives are particularly advantageous according to the invention:
(3S,8S,9S,10R,13R,14S,17R)-17-((R)-4,4-difluorobutan-2-yl)-10,13-dimethyl-2,3,4,7,8,9,10,11,12,13,14,15,16,17-tetradecahydro-1H-cyclopenta[a]phe-nanthren-3-ol,
(3S,8S,9S,10R,13S,14S,17R)-16-fluoro-10,13-dimethyl-17-((R)-6-methylhep-tan-2-yl)-2,3,4,7,8,9,10,11,12,13,14,15,16,17-tetradecahydro-1H-cyclo-penta[a]phenanthren-3-ol,
(3S,8S,9S,10R,13R,14S,17R)-17-((2R,6R)-7-fluoro-6-methylheptan-2-yl)-10,13-dimethyl-2,3,4,7,8,9,10,11,12,13,14,15,16,17-tetradecahydro-1H-cy-clopenta[a]phenanthren-3-ol,
(3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclopenta[a]phe-nanthren-3-yl 2-fluoroacetate,
(3S,8S,9S,10R,13S,14S,17R)-17-((2S,5R)-5-ethyl-3-fluoro-6-methylheptan-2-yl)-10,13-dimethyl-2,3,4,7,8,9,10,11,12,13,14,15,16,17-tetradecahydro-1H-cyclopenta[a]phenanthren-3-ol, or
(3S,8S,9S,10R,13R,14S,17R)-17-((2R,5S)-5-ethyl-4-fluoro-6-methylheptan-2-yl)-10,13-dimethyl-2,3,4,7,8,9,10,11,12,13,14,15,16,17-tetradecahydro-1H-cyclopenta[a]phenanthren-3-ol.

The Use of radiolabeled cholesterol derivatives according to the invention as well as their physiologically tolerated salts for the preparation of pharmaceutical drugs for the treatment of infectious diseases, particularly pneumonia or septic cardiomyopathy, but also other diseases associated with decreased cholesterol levels (hypercholesterinemia), e.g. Smith-Lemli-Opitz syndrome.

Pharmaceutical formulation are particularly preferred, which contain the radiolabeled cholesterol derivatives as well as their physiologically tolerated salts for the treatment of infectious diseases, particularly pneumonia or septic cardiomyopathy, but also other diseases associated with decreased cholesterol levels (hypercholesterinemia), e.g. Smith-Lemli-Opitz syndrome.

### Anti-bacterial properties

The liposomes exhibited at least one of two anti-infective properties:
1. Sequestration of virulence factors of gram-negative and gram-positive bacteria, e.g., pneumolysin, intermedilysin, aerolysin, and lipopolysaccharide.
2. Integration into the cell membrane, increasing resistance to bacterial virulence factors.

### Testing Procedures:

### 1. Sequestration of Virulence Factors:

Pneumolysin (Ply), the major virulence factor of S. pneumonia that was used here as a reference virulence factor, was incubated with liposomes containing Compounds 1 to 12, native cholesterol, or a mixture of both. As a reference, Ply was added to PBS without cholesterol or liposomes, and 0.5% Tween-20 in PBS was used as a positive control.

These mixtures were added to tubes containing a fixed number of erythrocytes isolated from human blood samples and incubated for 30 minutes with rocking before measuring hemolysis.

Hemolysis was quantified by removing intact erythrocytes through centrifugation and measuring the hemoglobin absorbance in the supernatant. The Tween-20 sample served as the maximum lysis control (100%), and the Ply in PBS acted as a positive control to validate that Ply causes hemolysis. The inhibition of hemolysis correlates with the toxin-sequestering anti-infective potency of the compounds.

### 2. Integration into the Cell Membrane:

Cells were incubated with liposomes containing Compounds 1 to 12, native cholesterol, or a mixture of both. After 30 minutes, the cells were washed, and Ply was added for 90 minutes.

Lactate dehydrogenase (LDH) activity in the supernatant was then quantified using commercial assays. As a reference, Ply was added to cells not treated with liposomes, and 1% Triton-X100 was used as a positive control.

LDH is released through Ply pores or from Triton-X100-lysed cells. Thus, the LDH activity in the supernatant correlates with Ply's pore-forming activity. A reduction in LDH activity indicates that the cells are protected against the crucial virulence factors that bacteria require to multiply, spread, and evade the immune system.

The use of isotope labeled cholesterol derivatives, as described in claims 1 and 2, and their physiologically tolerated salts, for the preparation of pharmaceutical drugs for treating infectious diseases such as pneumonia and sepsis. Additionally, these compounds can be used in diagnostics and personalized therapy for nanomedicines, primarily - but not exclusively - used in tumor treatments. This includes modifying approved nanoformulations (e.g., Ablecet, Ambisome, Depocyt, DaunoXome, Myocet, Epaxal, Inflexal V, Visudyne, Onpattro, Arikayce, Vyxeos CPX-321), adding a personalized therapeutic approach. Furthermore, these compounds serve as a general tool for replacing cholesterol in novel nanoformulations to achieve individualized drug monitoring.

The use of isotope labeled cholesterol derivatives, as described in claims 1 and 2, and their physiologically tolerated salts, for the preparation of pharmaceutical drugs for treating infectious diseases such as pneumonia and sepsis. Additionally, these compounds can be used in diagnostics and personalized therapy for nanomedicines, primarily - but not exclusively - used in tumor treatments. This includes modifying approved nanoformulations (e.g., Ablecet, Ambisome, Depocyt, DaunoXome, Myocet, Epaxal, Inflexal V, Visudyne, Onpattro, Arikayce, Vyxeos CPX-321), adding a personalized therapeutic approach. Furthermore, these compounds serve as a general tool for replacing cholesterol in novel nanoformulations to achieve individualized drug monitoring.

Easily accessible fluorinated cholesterol derivatives are for example: 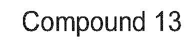 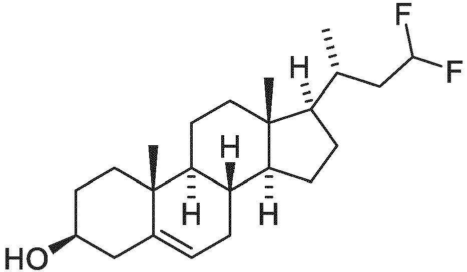 (3S,8S,9S,10R,13R,14S,17R)-17-((R)-4,4-difluorobutan-2-yl)-10,13-dimethyl-2,3,4,7,8,9,10,11,12,13,14,15,16,17-tetradecahydro-1H-cyclo-penta[a]phenanthren-3-ol (13), 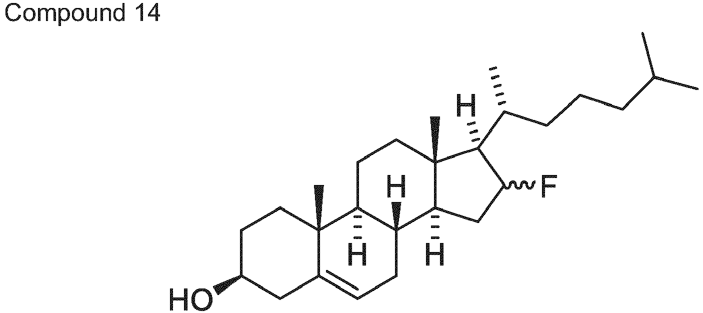 (3S,8S,9S,10R,13S,14S,17R)-16-fluoro-10,13-dimethyl-17-((R)-6-methylheptan-2-yl)-2,3,4,7,8,9,10,11,12,13,14,15,16,17-tetradecahydro-1H-cyclopenta[a]phenanthren-3-ol (14), 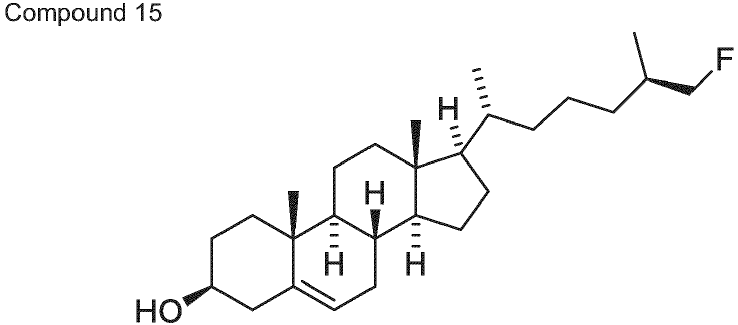 (3S,8S,9S,10R,13R,14S,17R)-17-((2R,6R)-7-fluoro-6-methylheptan-2-yl)-10,13-dimethyl-2,3,4,7,8,9,10,11,12,13,14,15,16,17-tetradecahydro-1H-cyclopenta[a]phenanthren-3-ol (15), or 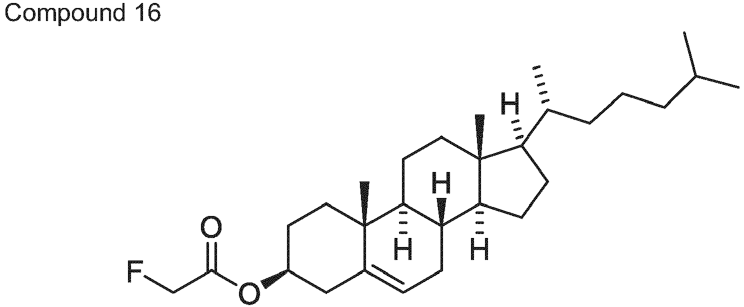 (3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclo-penta[a]phenanthren-3-yl 2-fluoroacetate (16).

Easily accessible fluorinated β-sitosterol derivatives are, for example: 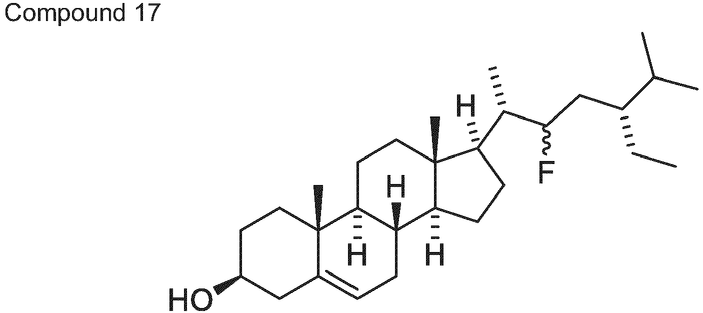 (3S,8S,9S,10R,13S,14S,17R)-17-((2S,5R)-5-ethyl-3-fluoro-6-methylheptan-2-yl)-10,13-dimethyl-2,3,4,7,8,9,10,11,12,13,14,15,16,17-tetradecahydro-1H-cyclopenta[a]phenanthren-3-ol (17), or 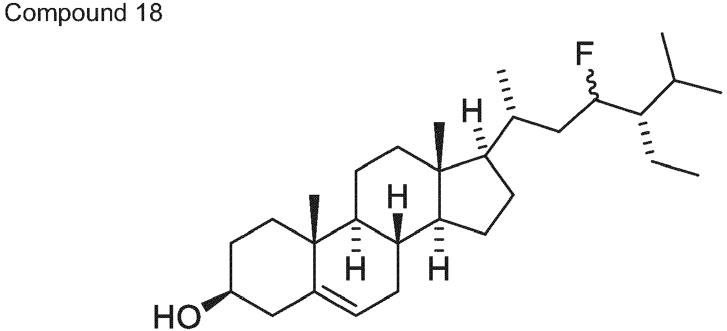 (3S,8S,9S,10R,13R,14S,17R)-17-((2R,5S)-5-ethyl-4-fluoro-6-methylheptan-2-yl)-10,13-dimethyl-2,3,4,7,8,9,10,11,12,13,14,15,16,17-tetradecahydro-1H-cyclopenta[a]phenanthren-3-ol (18).

### Experimental Procedures

Liposomes were formulated using the extrusion method. Dipalmitoylphospha-tidylcholine, used as a carrier lipid, was dissolved in chloroform along with native cholesterol and the novel compounds (Compounds 1 to 12). A lipid cake was created by evaporating the solvent under a constant nitrogen stream. This lipid cake was then hydrated in type-1 water using an ultrasonic bath maintained below the transition temperature of the lipids. The resulting suspension was extruded 9 to 13 times using a manual extruder equipped with 100 nm membranes. The liposomes were characterized by measuring their Zeta potential and hydrodynamic radius using dynamic light scattering.

### Synthesis of Compound 1, namely β-Sitosterol

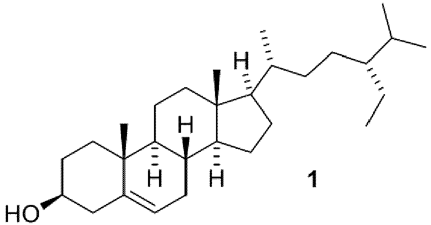

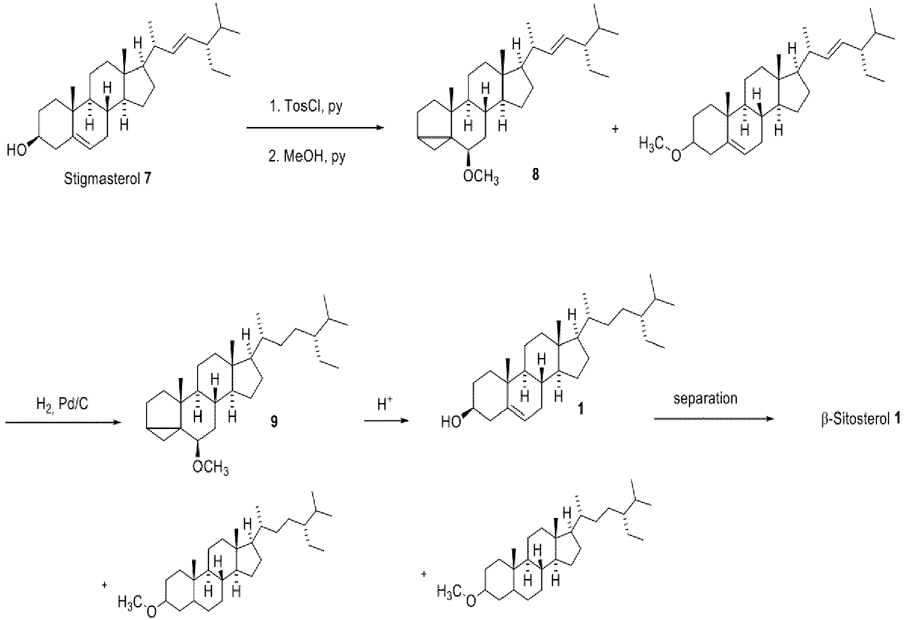

### Compound 8:

A solution of stigmasterol 7 (25.010 g, 57 mmol, 95% pure), 4-DMAP (0.7 g, 10 mol%) and tosyl chloride (23.0 g, 120 mmol) in pyridine (250 mL) was stirred at rt for 6 h. The solution was poured into 10% aq. sodium bicarbonate (1000 mL) and the precipitate was filtered, washed with water, dried and recrystallised from acetone to give the corresponding tosylate (30.6 g, 95 %) as white needles.

The tosylate (14.303 g, 25.2 mmol) and pyridine (6.1 mL, 3 eq.) were dissolved in anhydrous methanol and refluxed for 6 h. The solution was evaporated and extracted into ethyl acetate (400 mL) and washed with water (2× 300 mL). The organic layer was washed with saturated aq. sodium chloride (2 × 200 mL) and dried over magnesium sulfate. The residue was then purified and dried over magnesium sulfate. The residue was then purified by chromatography on silica gel using 5% ethyl acetate in hexane to give the i-stigmasterolmethyl ether 8 (8.211 g, 74 %) as an oily solid as a mixture (5 : 1) with stigmasterol methyl ether. This mixture was used unpurified in the next step.

### Compound 9:

To i-stigmasterolmethyl ether 8 (0.60 g, 1.4 mmol) in DCM/MeOH (5:1, 10 ml) under a nitrogen atmosphere were added Pd/C (0.15 g, 0.1 eq.) and K₂CO₃ (0.19 g, 1 eq.). The nitrogen atmosphere was replaced by hydrogen and the mixture was stirred for two days. The solids were removed by filtration with celite^{®} and the solution evaporated to give 9 (0.6 g, quant.), which was used without further processing.

### Compound 1:

### (3S,8S,9S,10R,13R,14S,17R)-17-((2R,5R)-5-ethyl-6-methylheptan-2-yl)-10,13-dimethyl-2,3,4,7,8,9,10,11,12,13,14,15,16,17-tetradecahydro-1H-cy-clopenta[a]phenanthren-3-ol

Compound 9 (0.90 g, 2.1 mmol) was dissolved in toluene (19 ml) and cooled to 0 °C prior to the addition of TFA (1 ml). After 10 minutes at 0 °C a solution of KOH in MeOH (10 %, 20 ml) was added and stirred for two minutes. Sat. NaCl (100 ml) was added and the product was extracted with ethyl acetate, dried using MgSO4 and evaporated. The residue was purified by flash chromatography on silica (eluent pentane / diethyl ether 3:1) to give β-Sitosterol 1 (0.80 g, 91 %).

### Synthesis of Compound 2

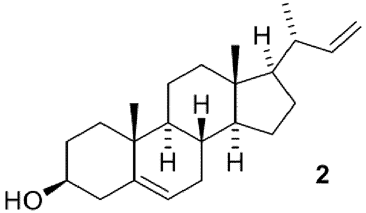

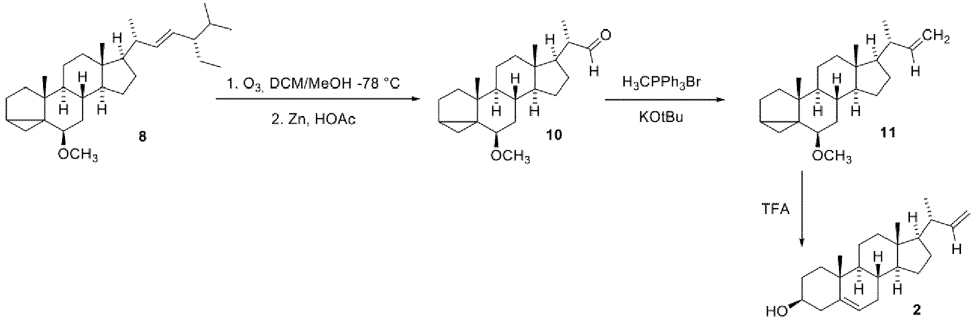

### Compound 10:

A solution of 8 (1.4 g, 3.27 mmol) in DCM/MeOH (2:1, 60 ml) was treated at - 78 °C with ozone for two hours. The ozone was removed by a stream of oxygen and the resulting mixture was treated with zinc (2 g) and acetic acid (5.5 ml) at room temperature for one hour. The mixture was evaporated, water was added (20 ml) and the product was extracted with ethyl acetate. The organic phase was washed with sat. NaHCO₃ and brine, dried and evaporated. The residue was purified by flash chromatography on silica (eluent pentane / diethyl ether 10:1) to give aldehyde 10 (0.17 g, 15 %).

### Compound 11:

Methyltriphenylphosphonium bromide (0.20 g, 0.6 mmol) in dry THF (10 ml) was treated with KOtBu (0.13 g, 1.2 mmol) for 30 min at ambient temperature. Then aldehyde 10 (0.16 g, 0.5 mmol) in THF (5 ml) was added and the mixture was stirred for one hour. After filtration through a short path of silica and evaporation, the olefin 11 (0.05 g, 31 %) was isolated by chromatography (pentane / ether 4:1).

### Compound 2:

### (3S,8S,9S,10R,13R,14S,17R)-17-((R)-but-3-en-2-yl)-10,13-dimethyl-2,3,4,7,8,9,10,11,12,13,14,15,16,17-tetradecahydro-1H-cyclopenta[a]phe-nanthren-3-ol

Compound 11 (0.15 g, 0.4 mmol) was dissolved in toluene (9.5 ml) and cooled to 0 °C prior to the addition of TFA (0.5 ml). After 10 minutes at 0 °C a solution of KOH in MeOH (10%, 10 ml) was added and stirred for two minutes. Sat. NaCl (20 ml) was added and the product was extracted with ethyl acetate, dried using MgSO4 and evaporated. The residue was purified by flash chromatography on silica (eluent pentane / diethyl ether 3:1) to give 2 (0.1 g, 70%).

### Synthesis of Compounds 3, 4 and 5

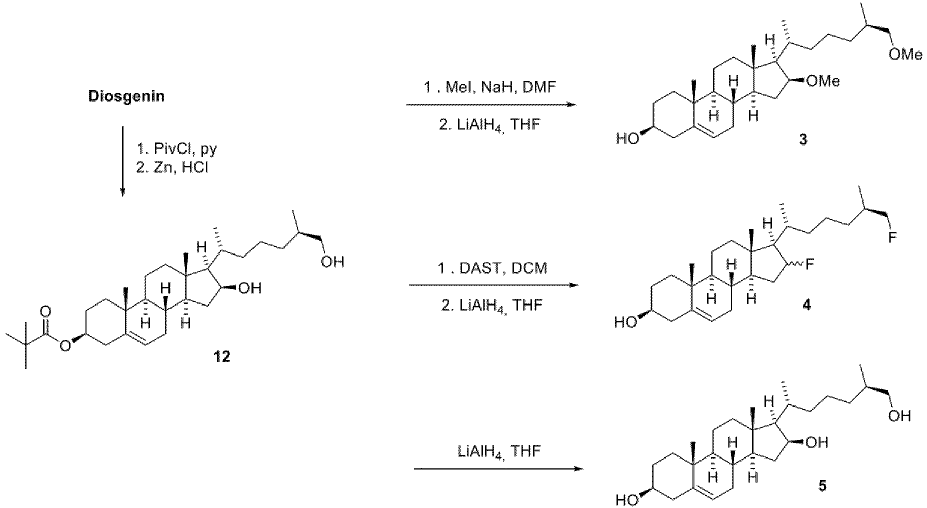

### Compound 12:

Diosgenin (13.00 g, 31.38 mmol) was dissolved in pyridine (110 ml). Pivaloyl chloride (4.6 ml, 37.65 mmol, 1.2 eq) was added dropwise and the reaction mixture was stirred at room temperature for 6 h. After the reaction was over, the mixture was diluted with toluene (250 ml). The organic phase was washed with 1 M HCl solution (3 × 150 ml), saturated aqueous NH₄Cl solution (100 ml), water (100 ml), sat. NaCl solution and dried over Na₂SO₄. The toluene was concentrated on a rotary evaporator until the first solid formed. Concentration was stopped and refluxed until solid had dissolved again. The solution was left to stand at room temperature overnight, where upon Piv-diosgenin crystallized as fine white needles. The crystals were filtered off and washed with some ice-cold ethanol (100 mL). The filtrate was concentrated on a rotary evaporator and dissolved in hot toluene. The solution was left to stand over night at room temperature. The crystals were filtered off and the second crop was washed with some ice-cold ethanol. After drying under high vacuum, a total of 12.66 g (25.38 mmol, 81 %) of Piv-diosgenin was obtained.

Piv-diosgenin (12.66 g, 25.38 mmol) and 240 g zinc powder were suspended in a mixture of n-propanol/ toluene (3:1, 500 ml) and heated to 75 °C. With vigorous stirring, fuming hydrochloric acid (37 %, 240 ml) was added slowly over 1 h and subsequently the mixture was heated to 80 °C for 3. After the reaction had ended, the solids (153 g of zinc were recovered) were filtered off and washed with toluene (150 ml) and water (150 ml) (153 g of zinc were recovered). The phases were separated and the organic phase washed with water (2 × 150 mL). The united aqueous phases were extracted with toluene (100 ml). The combined organic phases were washed with saturated NaCl solution (200 ml), dried over Na₂SO₄ and concentrated on a rotary evaporator until the first solids formed. The concentration was stopped and refluxed until the solid had dissolved. The solution was left overnight at room temperature and Piv-diosgenin-diol 12 crystallized as a fine white solid. The crystals were filtered off and washed with ice-cold toluene (200 ml). After drying under high vacuum, 10.33 g (81 %, 20.55 mmol) of Piv-diosgenin-diol 12 were obtained.

¹H NMR (400 MHz, Chloroform-d) δ 5.30 (d, J = 5.0 Hz, 1H), 4.73- 4.65 (m, 1H), 4.48-4.28 (m, 1H),3.47 - 3.33 (m, 2H), 2.23 (s, 2H), 1.93-11.88 (m, 3H), 1.81 - 1.73 (m, 2H),1.65-1.55(m,3H), 1.32 - 1.28 (m, 1H), 1.21-1.11 (m, 17H), 1.07 (s, 9H), 0.97 (s, 3H), 0.91 (d, J = 6.7 Hz, 3H), 0.84 (d, J = 6.7 Hz, 3H), 0.82 (s, 3H).

### Compound 3:

### (3S,8S,9S,10R,13S,14S,16S,17R)-16-methoxy-17-((2R)-7-methoxy-6-methylheptan-2-yl)-10,13-dimethyl-2,3,4,7,8,9,10,11,12,13,14,15,16,17-tetradecahydro-1H-cyclopenta[a]phenanthren-3-ol

Diol 12 (1g, 1,99 mmol) was solubilized in 25 mL of DMF under inert atmosphere, then methyl iodide (1.07 ml, 21 mmol, 10.5 eq.) and sodium hydride (375 mg, 14.91 mmol, 4.5 eq.) were added and stirred for 3h hours at 50 °C. Then sodium hydride (375 mg, 14.91 mmol, 4.5 eq.) was added again and the mixture stirred 1h at 50°C. Water (50 mL) was added and the mixture was extracted with diethyl ether (3 × 25 mL) and dried over Na₂SO₄. The product was purified by chromatography column in condition pentane/ diethyl ether (10:1) to give the bis-methylated compound (0.720 g, 1.35 mmol, 68%).

¹H NMR (400 MHz, Chloroform-d) δ 5.37 (d, J = 5.0 Hz, 1H), 4.68 - 4.46 (m, 1H), 3.81 - 3.59 (m, 1H), 3.33 (s, 3H), 3.24 (dd, J = 9.1, 5.9 Hz, 1H), 3.18 (s, 3H), 3.14 (dd, J = 9.0, 6.9 Hz, 1H), 2.34 - 2.26 (m, 2H), 2.15 - 2.04 (m, 1H), 2.04 - 1.93 (m, 2H), 1.92 - 1.74 (m, 3H), 1.76 - 1.59 (m, 3H), 1.54 (s, 3H), 1.53 - 1.26 (m, 12H), 1.18 (s, 9H), 1.03 (s, 3H), 0.90 (d, J = 6.8 Hz, 6H), 0.84 (s, 3H).

The dimethyl ether (586 mg, 1.10 mmol, 1 eq.) was solubilized in anhydrous THF (10mL) and slowly added to à suspension of LiAlH₄ (125 mg, 3.30 mmol, 3 eq.) in 10 mL of anhydrous THF. The mixture were stirred 3.5 h at room temperature. Then 10 ml of water was added to quench the reaction.

50 ml of aqueous potassium sodium tartrate solution was added and the mixture was extracted with ethyl acetate (3 × 50 mL). The organic phase was dried over Na2SO4 and purified by silica column pentane/diethyl ether (1:1) to give bis-methyl cholesterol 3 (0.359 g, 0.8042 mmol, 73 %).

¹H NMR (400 MHz, Chloroform-d) δ 5.35 (d, J = 5.2 Hz, 1H), 3.73 - 3.64 (m, 1H), 3.59 - 3.48 (m, 1H), 3.33 (s, 3H), 3.24 (dd, J = 9.1, 5.9 Hz, 1H), 3.27 - 3.11 (m, 3H), 3.14 (dd, J = 9.1, 6.9 Hz, 1H), 2.33 - 2.17 (m, 2H), 2.10 (dt, J = 12.8, 7.4 Hz, 1H), 2.04 - 1.95 (m, 2H), 1.89 - 1.78 (m, 3H), 1.76 - 1.66 (m, 1H), 1.55 (s, 3H), 1.50 - 1.03 (m, 15H), 1.01 (s, 3H), 0.90 (d, J = 6.8 Hz, 6H), 0.84 (s, 3H).

### Compound 4:

### (3S,8S,9S,10R,13S,14S,17R)-16-fluoro-17-((2R)-7-fluoro-6-methylhep-tan-2-yl)-10,13-dimethyl-2,3,4,7,8,9,10,11,12,13,14,15,16,17-tetradecahy-dro-1H-cyclopenta[a]phenanthren-3-ol

A solution of diol 12 (0.2 g, 0.4 mmol) in DCM (10 ml) was cooled to -78 °C and DAST (0.19 g, 3 eq.) was added. The mixture was slowly warmed to room temperature and stirred overnight. After hydrolysis with sat. NaHCO3, extraction with DCM, drying with MgSO4 and evaporation, the crude difluoro compound was used for the next step without purification.

The crude difluoride was solubilized in anhydrous THF (10 mL) and slowly added to a suspension of LiAlH₄ (50 mg, 1.30 mmol) in 10 mL of anhydrous THF. The mixture were stirred 5 h at room temperature. Then 10 ml of water was added to quench the reaction. 50 ml of aqueous potassium sodium tartrate solution was added and the mixture was extracted with ethyl acetate (3 × 50mL). The organic phase was dried over Na₂SO₄ and purified by silica column pentane/diethyl ether (1:1) to give compound 4 (0.04 g, 24 % over 2 steps).

Based on a modified procedure the desired mono- and difluorides can be also available by the use of 18F-DAST.

### Compound 5:

### (3S,8S,9S,10R,13S,14S,16S,17R)-17-((2R)-7-hydroxy-6-methylheptan-2-yl)-10,13-dimethyl-2,3,4,7,8,9,10,11,12,13,14,15,16,17-tetradecahydro-1H-cyclopenta[a]phenanthrene-3,16-diol

A solution of diol 12 (0.2 g, 0.4 mmol) was solubilized in anhydrous THF (10mL) and slowly added to a suspension of LiAlH₄ (50 mg, 1.30 mmol) in 10 mL of anhydrous THF. The mixture was stirred overnight at room temperature. Then 10 ml of water was added to quench the reaction. 50 ml of aqueous potassium sodium tartrate solution was added and the mixture was extracted with ethyl acetate (3 × 50mL). The organic phase was dried over Na2SO4 and purified by silica column diethyl ether to give compound 5 (0.06 g, 36%).

### Synthesis of Compound 6

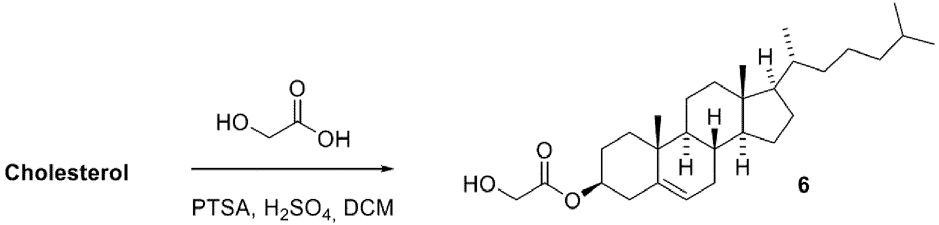

### Compound 6:

### (3S,8S,9S,10R,13R,14S,17R)-10,13-dimethyl-17-((R)-6-methylheptan-2-yl)-2,3,4,7,8,9,10,11,12,13,14,15,16,17-tetradecahydro-1H-cyclo-penta[a]phenanthren-3-yl 2-hydroxyacetate

Cholesterol (284.12 mg, 0.7354 mmol, 1 eq.) was solubilized in 5 mL of DCM, then hydroxy acetic acid (221.39 mg, 2.911 mmol, 4 eq.). The mixture was stirred 15min at room temperature, then PTSA was added and the mixture was stirred 6 h at reflux. One drop of conc. H₂SO₄ was added for completion of the reaction by continuing for 1 hour at reflux. Then 30 mL of brine solution was added and extracted with diethyl ether (3 X3 0mL). Organic phase was dried over Na2SO4, concentrated, and purified by silica column pentane/diethyl ether (5:1) to give ester 6 (0.170 g, 0.382 mmol, 52%).

¹H NMR (400 MHz, Chloroform-d) δ 5.41 - 5.37 (m, 1H), 4.78 - 4.70 (m, 1H), 4.12 (s, 2H), 2.37 - 2.33 (m, 2H), 2.06 - 1.74 (m, 8H), 1.22 - 1.06 (m, 16H), 1.02 (s, 3H), 1.00 - 0.94 (m, 3H), 0.91 (d, J = 6.6 Hz, 3H), 0.87 (d, J = 1.9 Hz, 3H), 0.85 (d, J = 1.8 Hz, 3H), 0.68 (s, 3H).

## Claims

1. Isotope labeled cholesterol derivatives of general formula I 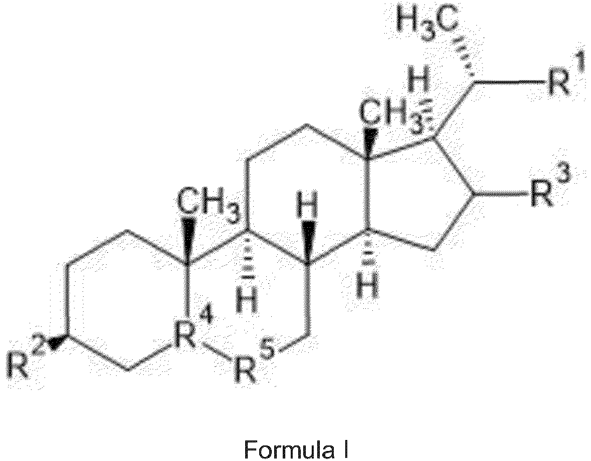 wherein
R¹ is -CH=CH₂, .-CH₂-CHX₂, -CH₂-CH₂-CH₂-CH(CH₃)₂, -CH₂-CH₂-CH₂-CH(CH₃)(CH₂X), -CH₂-CH₂-CH₂-CH(CH₃)(CH₂OCH₃), -CH₂-CH₂-CH₂-CH(CH₃)(CH₂OH), -CH₂-CH₂-CH(CH₂CH₃)(CH(CH₃)₂), -CHF-CH₂-CH(CH₂CH₃)(CH(CH₃)₂), or -CH₂-CHX-CH(CH₂CH₃)(CH(CH₃)₂),
R² is -OH, -OCH₃, -O-CO-CH₂OH, -O-CO-CH₂X, -O-CO-CHX₂, -O-CO-CX₃, or stealth polymers like polyethylene glycol (PEG), Poly(ethylene glycol) monomethyl ether (mPEG), Polyoxazolines (Pox), betaines and others,
R³ is -H, -F, -I, -D, -OH, or -OCH₃
R⁴ is -CH or -CD,
R⁵ is -CH₂, -CHD or -CD₂,
R⁴ and R⁵ is -CH= CH₂-, -CH= CD₂-, -CH=CHD-, -CD=CH₂, -CD=CD₂-or -CD=CHD-,
X is F or I or D,
and wherein at least one of the groups R¹ to R³ contains radio labeled and/or labelled isotopes, namely ¹⁸F, and/or ¹³¹I, and/or ¹²⁴I, and/or deuterium isotope labeled derivatives.

2. Isotope labeled cholesterol derivatives according to claim 1, namely
(3S,8S,9S,10R,13R,14S,17R)-17-((R)-4,4-difluorobutan-2-yl)-10,13-dimethyl-2,3,4,7,8,9,10,11,12,13,14,15,16,17-tetradecahydro-1H-cyclo-penta[a]phenanthren-3-ol,
(3S,8S,9S,10R,13S,14S,17R)-16-fluoro-10,13-dimethyl-17-((R)-6-methylheptan-2-yl)-2,3,4,7,8,9,10,11,12,13,14,15,16,17-tetradecahydro-1H-cyclopenta[a]phenanthren-3-ol,
(3S,8S,9S,10R,13R,14S,17R)-17-((2R,6R)-7-fluoro-6-methylheptan-2-yl)-10,13-dimethyl-2,3,4,7,8,9,10,11,12,13,14,15,16,17-tetradecahydro-1H-cyclopenta[a]phenanthren-3-ol,
(3S,8S,9S, 10R,13R,14S,17R)-10,13-dimethyl-17-((R)-6-methylheptan-2-yl)-2,3,4,7,8,9,10,11,12,13,14,15,16,17-tetradecahydro-1H-cyclo-penta[a]phenanthren-3-yl 2-fluoroacetate,
(3S, 8S ,9S, 10 R, 13S, 14S, 17R)-17-((2S,5R)-5-ethyl-3-fluoro-6-methylheptan-2-yl)-10,13-dimethyl-2,3,4,7,8,9,10,11,12,13,14,15,16,17-tetradecahydro-1H-cyclopenta[a]phenanthren-3-ol, or
(3S,8S,9S,10R,13R,14S,17R)-17-((2R,5S)-5-ethyl-4-fluoro-6-methylheptan-2-yl)-10,13-dimethyl-2,3,4,7,8,9,10,11,12,13,14,15,16,17-tetradecahydro-1H-cyclopenta[a]phenanthren-3-ol.

3. Use of radiolabeled cholesterol derivatives according to claim 1 or 2 as well as their physiologically tolerated salts for the preparation of pharmaceutical drugs for the treatment of infectious diseases, particularly pneumonia or septic cardiomyopathy, but also other diseases associated with decreased cholesterol levels (hypercholesterinemia), e.g. Smith-Lemli-Opitz syndrome.

4. Pharmaceutical formulation containing radiolabeled cholesterol derivatives according to claim 1 or 2 as well as their physiologically tolerated salts for the treatment of infectious diseases, particularly pneumonia or septic cardiomyopathy, but also other diseases associated with decreased cholesterol levels (hypercholesterinemia), e.g. Smith-Lemli-Opitz syndrome.
